# EUROPÄISCHE PATENTSCHRIFT

(11) **EP 1 298 426 B1**
(45) Veröffentlichungstag und Bekanntmachung des Hinweises auf die Patenterteilung: **03.01.2007**
(21) Anmeldenummer: 02018618.5
(22) Anmeldetag: 20.08.2002
(51) Int. Cl.: G01N 1/12, C21C 5/46

(54) **Probennehmer für Schmelzen**
Sampling probe for melts
Sonde d'échantillonnage pour bains de fusion

(30) Priorität: 28.09.2001 DE 10148112
(43) Veröffentlichungstag der Anmeldung: 02.04.2003
(73) Patentinhaber: Heraeus Electro-Nite International N.V., 3530 Houthalen (BE)
(72) Erfinder: Knevels, Johan, 3690 Bree (BE)
(74) Vertreter: Kühn, Hans-Christian

(56) Entgegenhaltungen:
- EP-A- 1 126 036
- DE-A- 19 752 743
- DE-U- 7 405 180
- US-A- 5 057 149

## Beschreibung

Die Erfindung betrifft einen Probennehmer für Schmelzen, insbesondere für Schlacke, mit einem Körper, der einen Einlauf und eine Probenkammer mit Einlauföffnung aufweist, wobei die Einlauföffnung in einer Kammerwand angeordnet ist, die durch eine eine Öffnung aufweisende erste Metallplatte begrenzt ist.

Derartige Probennehmer sind aus DE 197 52 743 A1 sowie aus EP 1 126 036 A1 bekannt. Derartige Probennehmer ermöglichen eine effektive Entnahme von Schlackeschmelze aus einem Schmelzbad. Mit den bekannten Probennehmern kann es jedoch Probleme geben bei der Entnahme der Probe aus der Probenkammer.

Weitere Probennehmer sind aus US 5,057,149 und DE-GM 7 405 180 bekannt. Aus US 5,057,149 ist es bekannt, eine aus mehreren, sich vor bzw. während des Einlaufens der Metallschmelze in den Einlaufkanal einer Probenkammer auflösenden Schichten gebildete temporäre Schutzkappe anzuordnen. Vor dem Einlaufkanal in eine Probenkammer angeordnete Schutzkappen sind ebenfalls aus DE-GM 7 405 180 bekannt.

Aufgabe der vorliegenden Erfindung ist es, die bekannten Probennehmer zu verbessern und die Entnahme der Probe aus der Probenkammer zu vereinfachen.

Die Aufgabe wird erfindungsgemäß durch die Merkmale des Anspruchs 1 gelöst. Dadurch, dass an der ersten Metallplatte eine zweite Metallplatte flächig angeordnet ist, wobei die zweite Metallplatte eine Öffnung aufweist, die zusammen mit der Öffnung der ersten Metallplatte die Einlauföffnung bildet, ist die Probe der Probenkammer leicht zu entnehmen. Die beiden aneinander anliegenden Metallplatten lassen sich zumindest geringfügig gegeneinander bewegen. Darüber hinaus gewährleisten die beiden aneinander anliegenden Metallplatten eine bessere Wärmeisolation der Probe. Ein verringerter Wärmetransport von der Metallschmelze außerhalb der Probenkammer und der Probe selbst führt zu einer geringerer Anhaftung der Probe an der die Einlauföffnung umgebenden Metallplatte.

Zweckmäßigerweise sind Form und Größe der Öffnungen der beiden Metallplatten gleich und die beiden Öffnungen sind vorzugsweise zentrisch angeordnet. Dadurch kann die Schmelze gleichmäßig und ohne zusätzliche Wirbelbildung in die Probenkammer einlaufen. Vorteilhafterweise sind die Metallplatten aus Stahl gebildet. Sie sind jeweils etwa 1 mm dick.

Die Probenkammer ist vorteilhafterweise als Flachprobenkammer ausgebildet, wobei die der Einlauföffnung gegenüberliegende Wand der Probenkammer aus einer weiteren Metallplatte, insbesondere aus einer Stahlplatte, gebildet sein kann und wobei die Flachprobenkammer eine seitlich umlaufende, an die Metallplatte angrenzende Wand aufweisen kann, die ebenfalls aus Metall, insbesondere aus Stahl, gebildet sein kann. Die umlaufende Wand kann an ihrer der Probenkammer zugewandten Oberfläche eine ringförmig umlaufende Nut aufweisen.

Es ist von Vorteil, dass vor den beiden die Öffnungen aufweisenden Metallplatten eine Vorkammer in den Körper angeordnet ist, in der die Schlacke vor Einlauf in die Probenkammer gesammelt wird. Die Vorkammer kann trichterförmig ausgebildet sein, d. h., sie weist an ihrem der Einlauföffnung gegenüberliegenden Ende eine größere Querschnittsfläche auf als an dem der Einlauföffnung zugewandten Ende. Dabei ist die der Einlauföffnung gegenüberliegende größere Querschnittsfläche als Öffnung für die Probennahme (Probennahmeöffnung) ausgebildet. Zweckmäßigerweise ist der die Probenkammer und die Vorkammer umgebende Körper aus Gießereisand gebildet. Der Körper kann entweder an einem Träger angeordnet sein, so dass der Probennehmer als Tauchprobennehmer verwendet wird oder er weist an seinem der Probennahmeöffnung gegenüberliegenden Ende eine Stellfläche auf oder ist in bekannter Weise so gehaltert, dass die Probennahmeöffnung den oberen Abschluss des Körpers bildet. In diesem Fall kann eine Schlackenprobe von oben in die Vorkammer eingegossen werden. Sie läuft dann von oben in die Probenkammer.

Nachfolgend wird ein Ausführungsbeispiel der Erfindung anhand einer Zeichnung näher erläutert.

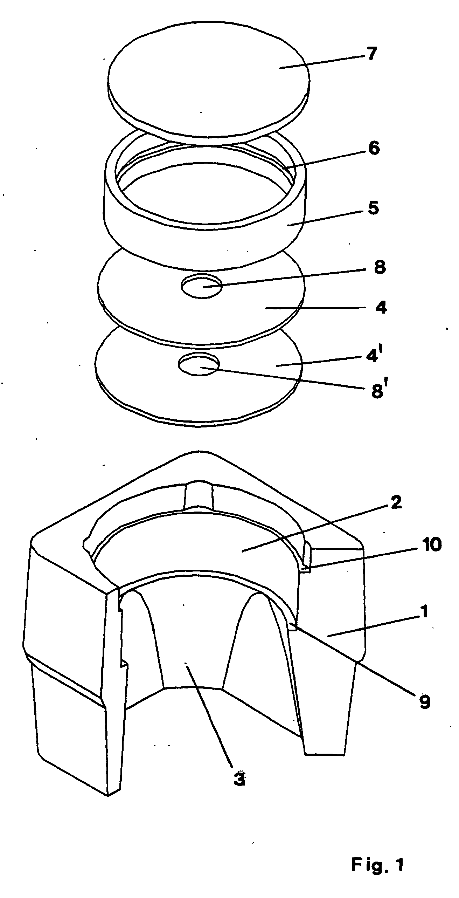

In der Zeichnung zeigt
- Figur 1: die Einzelteile des Probennehmers, im Schnitt,

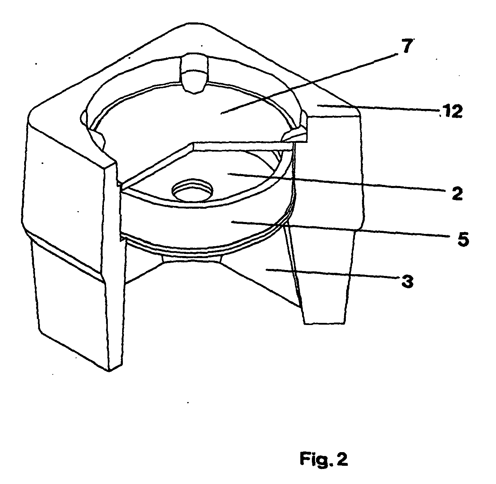

- Figur 2: den Probennehmer, im Schnitt, montiert und

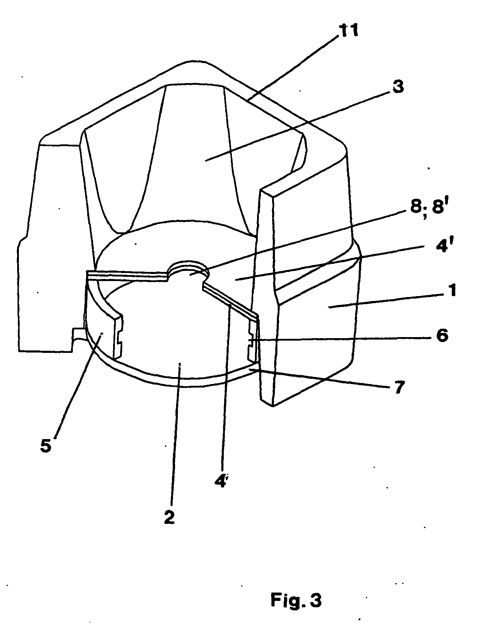

- Figur 3: einen Probennehmer in Betriebsstellung, im Schnitt.

Der Körper 1 aus Gießereisand weist eine Probenkammer 2 zur Bildung von Flachproben sowie eine trichterartige Vorkammer 3 auf. Die Probenkammer 2 ist zur Vorkammer 3 hin durch eine erste Metallplatte 4 und eine zweite Metallplatte 4' begrenzt. Seitlich ist sie begrenzt durch eine umlaufende Wand 5 aus einem Stahlring, der an seiner inneren Oberfläche eine umlaufende Nut 6 aufweist. Die dritte Metallplatte 7, die den beiden Metallplatten 4, 4' gegenüberliegend angeordnet ist, bildet den Abschluss der Probenkammer. In den Metallplatten 4, 4' ist jeweils eine Öffnung 8, 8' vorgesehen, die zentrisch angeordnet sind und als kreisförmige Bohrung ausgebildet sind. Die Öffnungen 8, 8' bilden gemeinsam die Einlauföffnung in die Probenkammer 2.

Die Montage eines derartigen Probennehmers ist relativ einfach, die Einzelteile werden einfach von einer Seite her in den Körper eingesetzt, wobei die Metallplatten 4;4' und 7 an jeweils einer umlaufenden Anschlagschulter 9, 10 anliegen. Die Metallplatten 4;7 liegen an der umlaufenden Wand 5 an.

Die Vorkammer 3 weist mit ihrem der Einlauföffnung gegenüberliegenden Ende 11 ihre größte Querschnittsfläche auf. In den dadurch gebildeten Trichter kann die Schmelze mittels eines Gießlöffels von oben in die Vorkammer 3 eingegossen werden, wobei der Körper 1 mit seiner Auflagefläche 12 auf einem feuerfesten Untergrund steht.

## Patentansprüche

1. Probennehmer für Schmelzen, insbesondere für Schlacke, mit einem Körper, der einen Einlauf und eine Probenkammer mit Einlauföffnung aufweist, wobei die Einlauföffnung in einer Kammerwand angeordnet ist, die durch eine eine Öffnung aufweisende erste Metallplatte begrenzt ist, **dadurch gekennzeichnet, dass** an der ersten Metallplatte (4) eine zweite Metallplatte (4') flächig angeordnet ist, wobei die zweite Metallplatte (4') eine Öffnung (8') aufweist, die zusammen mit der Öffnung (8) der ersten Metallplatte (4) die Einlauföffnung (8;8') bildet.

2. Probennehmer nach Anspruch 1, **dadurch gekennzeichnet, dass** Form und Größe der Öffnungen (8;8') der beiden Metallplatten (4;4') gleich sind.

3. Probennehmer nach Anspruch 1 oder 2, **dadurch gekennzeichnet, dass** die Metallplatten (4;4') aus Stahl gebildet sind.

4. Probennehmer nach einem der Ansprüche 1 bis 3, **dadurch gekennzeichnet, dass** die Probenkammer (2) als Flachprobenkammer ausgebildet ist.

5. Probennehmer nach Anspruch 4, **dadurch gekennzeichnet, dass** die der Einlauföffnung gegenüberliegende Wand der Probenkammer (2) aus einer Metallplatte (7), insbesondere aus einer Stahlplatte, gebildet ist.

6. Probennehmer nach Anspruch 4 oder 5, **dadurch gekennzeichnet, dass** die Flachprobenkammer eine seitlich umlaufende, an die Metallplatten (4;4') angrenzende Wand (5) aufweist, die aus Metall, insbesondere aus Stahl, gebildet ist.

7. Probennehmer nach Anspruch 6, **dadurch gekennzeichnet, dass** die seitlich umlaufende Wand (5) an ihrer der Probenkammer (2) zugewandten Oberfläche eine ringförmig umlaufende Nut (6) aufweist.

8. Probennehmer nach einem der Ansprüche 1 bis 7, **dadurch gekennzeichnet, dass** vor den beiden die Öffnungen (8;8') aufweisenden Metallplatten (4;4') eine Vorkammer (3) in dem Körper (1) angeordnet ist.

9. Probennehmer nach Anspruch 8, **dadurch gekennzeichnet, dass** die Vorkammer (3) an ihrem der Einlauföffnung gegenüberliegenden Ende (11) eine größere Querschnittsfläche aufweist, als an dem der Einlauföffnung zugewandten Ende.

10. Probennehmer nach Anspruch 9, **dadurch gekennzeichnet, dass** die Vorkammer (3) eine Probennahmeöffnung aufweist, die durch die größere Querschnittsfläche gebildet ist.

11. Probennehmer nach einem der Ansprüche 1 bis 10, **dadurch gekennzeichnet, dass** der Körper (1) aus Gießereisand gebildet ist.

## Claims

1. Sampler for melts, especially for slag, with a body comprising an inlet and a sampling chamber with inlet opening, the inlet opening being arranged in a chamber wall which is limited by a first metal plate comprising an opening, **characterized in that** - on the first metal plate (4) - a second metal plate (4') is planarly provided, with the second metal plate (4') comprising an opening (8') which, together with the opening (8) of the first metal plate (4), forms the inlet opening (8;8').

2. Sampler according to claim 1, **characterized in that** shape and size of the openings (8;8') of the two metal plates (4;4') are identical.

3. Sampler according to claim 1 or 2, **characterized in that** the metal plates (4;4') are made of steel.

4. Sampler according to any one of the claims 1 to 3, **characterized in that** the sampling chamber (2) is designed as a flat sampling chamber.

5. Sampler according to claim 4, **characterized in that** the wall of the sampling chamber (2) opposite the inlet opening is made of a metal plate (7), especially a steel plate.

6. Sampler according to claim 4 or 5, **characterized in that** the flat sampling chamber comprises a laterally circumferential wall (5) adjoining the metal plates (4;4') which is made of metal, especially of steel.

7. Sampler according to claim 6, **characterized in that** the laterally circumferential wall (5) comprises, on its surface facing the sampling chamber (2), a ring-shaped circumferential groove (6).

8. Sampler according to any one of the claims 1 to 7, **characterized in that**, in front of the two metal plates (4;4') comprising the openings (8;8'), an antechamber (3) is provided in the body (1).

9. Sampler according to claim 8, **characterized in that** the antechamber (3), on its end (11) opposite the inlet opening, has a larger cross-sectional area than on the end facing the inlet opening.

10. Sampler according to claim 9, **characterized in that** the antechamber (3) comprises a sampling opening which is formed by the larger cross-sectional area.

11. Sampler according to any one of the claims 1 to 10, **characterized in that** the body (1) is made of foundry sand.

## Revendications

1. Échantillonneur de fontes, en particulier de laitier, avec un corps qui comporte une entrée et une chambre à échantillons avec un orifice d'entrée, l'orifice d'entrée étant situé dans une paroi de la chambre qui est délimitée par une première plaque de métal présentant une ouverture, **caractérisé en ce qu'**une deuxième plaque de métal (4') est disposée de manière plane sur la première plaque de métal (4), la deuxième plaque de métal (4') présentant une ouverture (8') qui, conjointement avec l'ouverture (8) de la première plaque de métal (4), constitue l'orifice d'entrée (8;8').

2. Échantillonneur selon la revendication 1, **caractérisé en ce que** la forme et la taille des ouvertures (8;8') des deux plaques de métal (4;4') sont identiques.

3. Échantillonneur selon la revendication 1 ou 2, **caractérisé en ce que** les plaques de métal (4;4') sont constituées d'acier.

4. Échantillonneur selon l'une des revendications 1 à 3, **caractérisé en ce que** la chambre à échantillons (2) se présente sous la forme d'une chambre à échantillons plate.

5. Échantillonneur selon la revendication 4, **caractérisé en ce que** la paroi de la chambre à échantillons (2) opposée à l'orifice d'entrée est constituée d'une plaque de métal (7), en particulier d'une plaque en acier.

6. Échantillonneur selon la revendication 4 ou 5, **caractérisé en ce que** la chambre à échantillons plate comporte une paroi (5) périphérique latérale adjacente aux plaques de métal (4;4'), laquelle est constituée de métal, en particulier d'acier.

7. Échantillonneur selon la revendication 6, **caractérisé en ce que** la paroi périphérique latérale (5) comporte, sur sa surface tournée vers la chambre à échantillons (2), une rainure périphérique annulaire (6).

8. Échantillonneur selon l'une des revendications 1 à 7, **caractérisé en ce qu'**une préchambre (3) est située dans le corps (1) devant les deux plaques de métal (4;4') présentant les ouvertures (8;8').

9. Échantillonneur selon la revendication 8, **caractérisé en ce que** la préchambre (3), à son extrémité (11) opposée à l'orifice d'entrée, présente une surface de section transversale plus grande qu'à l'extrémité tournée vers l'orifice d'entrée.

10. Échantillonneur selon la revendication 9, **caractérisé en ce que** la préchambre (3) comporte un orifice d'échantillonnage qui est constitué par la surface de section transversale plus grande.

11. Échantillonneur selon l'une des revendications 1 à 10, **caractérisé en ce que** le corps (1) est constitué de sable de fonderie.
